# EUROPÄISCHE PATENTSCHRIFT

(11) **EP 0 668 905 B1**
(45) Veröffentlichungstag und Bekanntmachung des Hinweises auf die Patenterteilung: **09.09.1998**
(21) Anmeldenummer: 93924565.0
(22) Anmeldetag: 02.11.1993
(51) Int. Cl.: C11D 11/02, B01D 1/18, C11D 11/00

(54) **VERFAHREN ZUR ERLEICHTERTEN REINIGUNG VON WERTSTOFFEN UND/ODER WERTSTOFFGEMISCHEN AUS DEM BEREICH DER NETZ-, WASCH- UND/ODER REINIGUNGSMITTEL SOWIE ZUGEHÖRIGER WERTSTOFFE**
PROCESS FOR THE EASIER PURIFICATION OF VALUABLE MATERIALS AND/OR THEIR MIXTURES IN THE FIELD OF WETTING, WASHING AND/OR CLEANING AGENTS AND RELATED SUBSTANCES
PROCEDE D'EPURATION SIMPLIFIEE DE MATIERES UTILES ET/OU DE MELANGES DE TELLES MATIERES APPARTENANT A LA GAMME DES PRODUITS MOUILLANTS ET DES PRODUITS DE LAVAGE ET/OU DE NETTOYAGE, ET MATIERES UTILES CONNEXES

(30) Priorität: 11.11.1992 DE 4237934
(43) Veröffentlichungstag der Anmeldung: 30.08.1995
(73) Patentinhaber: Henkel Kommanditgesellschaft auf Aktien, 40191 Düsseldorf (DE)
(72) Erfinder: RÄHSE, Wilfried, D-40589 Düsseldorf (DE); FUES, Johann, Friedrich, D-41516 Grevenbroich (DE); SCHMID, Karl-Heinz, D-40822 Mettmann (DE); PAATZ, Kathleen, D-40593 Düsseldorf (DE)
(86) Internationale Anmeldenummer: EP9303047
(87) Internationale Veröffentlichungsnummer: WO9411486

(56) Entgegenhaltungen:
- WO-A-92/05849
- DE-A- 2 622 520
- DE-A- 3 447 867

## Beschreibung

### Gebiet der Erfindung

Die Erfindung betrifft ein vereinfachtes Verfahren zur Verbesserung des Reinheitsgrades und dabei insbesondere der Beschaffenheit von Farbe und Geruch in Wertstoffen und Wertstoffgemischen aus dem Bereich der Netz-, Wasch- und/oder Reinigungsmittel sowie zugehöriger Wertstoffe, beispielsweise aus dem Gebiet der Textilausrüstung. Die Erfindung will dabei insbesondere eine erleichterte Möglichkeit schaffen, den Geruch und/oder die Farbe der angegebenen Wertstoffe beziehungsweise Wertstoffgemische substantiell zu verbessern ohne an die bisher üblichen aufwendigen Reinigungsprozesse gebunden zu sein. Der Gedanke der erfindungsgemäßen Reinigung ist dabei aber nicht auf solche sensorisch wahrnehmbaren Begleitstoffe eingeschränkt, auch andere unerwünschte Begleitstoffe aus der Herstellung und/oder Verarbeitung von Wertstoffen beziehungsweise Wertstoffgemischen der angegebenen Art können durch das erfindungsgemäße Reinigungsverfahren erfaßt und von dem letztlich zu gewinnenden Gut abgetrennt werden.

### Stand der Technik

Die der Erfindung zugrundeliegende Problematik wird im nachfolgenden anhand von Wertstoffen aus dem Gebiet der textilen Wasch- und Reinigungsmittel geschildert, sie ist hierauf aber nicht eingeschränkt.

Es ist allgemeines Wissen, daß Textilwaschmittel oder in solchen Mitteln zum Einsatz kommende Komponenten vergleichsweise hohen Anforderungen an die Reinheit genügen müssen. Besondere Bedeutung kommt dabei den vom Endverbraucher sensorisch wahrzunehmenden Anforderungen an den Geruch und die Farbe des Waschmittels beziehungsweise Waschmittelgemisches zu. Das beispielsweise als rieselfähiges Feststoffmaterial vertriebene Waschmittel soll möglichst hellfarbig, aber besten rein-weiß sein. Ein gebräuntes oder grau verfärbtes Produkt erweckt den Eindruck einer minderwertigen, schmutzbeladenen Ware. Fast noch wichtiger ist der Eigengeruch des Wertstoffes beziehungsweise Wertstoffgemisches. Ein dumpfer talgartiger Geruch führt zur instinktiven Ablehnung. Erschwert ist dieser Aspekt für die Produktherstellung aus der Tatsache, daß Hilfsstoffe der hier genannten Art häufig über lange Zeiträume verpackt in fest verschlossenen Gefäßen unter variablen Außenbedingungen gelagert werden müssen und beim Öffnen der Verpackung durch den Verbraucher eine in diesem Moment sehr starke geruchs-sinnliche Wahrnehmung durch den Verbraucher stattfindet. Dumpfe, talgige, ranzige oder in anderer Weise scharfe Geruchseindrücke führen zur instinktiven Ablehnung, obschon eine objektiv feststellbare Beeinträchtigung der Produkteigenschaften nur in den seltensten Fällen gegeben ist.

Die mit der Herstellung von Wertstoffen beziehungsweise Wertstoffgemischen der erfindungsgemäß betroffenen Art befaßte Fachwelt ist darüber hinaus mit weiterführenden Problemen der Reinheit konfrontiert, die - ebenso wie die zuvor geschilderten Faktoren - beträchtlichen Aufwand zur Reinigung und Reindarstellung der Wertstoffe beziehungsweise Wertstoffgemische erfordern. Lediglich beispielhaft sei hier auf die Problematik der vollständigen Entfernung von Alkylenoxid-Resten (EO und/oder PO) sowie Dioxan aus den durch Alkoxylierung gewonnenen Wertstoffen von beispielsweise niotensidischem beziehungsweise anionischem Charakter verwiesen.

Erschwerend kommt hier der nachfolgende Gesichtspunkt zum Tragen: Wesentliche Wertstoffe aus dem erfindungsgemäß betroffenen Arbeitsgebiet sind entweder naturstoffbasiert oder aber durch Synthese aus Erdöl-basierten Rohstoffen gewonnen. Die Herstellung von Tensiden erfolgt in mehrstufigen Verfahren, die in der jeweiligen Arbeitsstufe zwar optimierte Ergebnisse bezüglich der gewünschten Komponente liefern, gleichwohl auch zur Entstehung von unerwünschten Begleitstoffen - gegebenenfalls in Spurenmengen - führen. Am Beispiel einiger aniontensidischer Komponenten und einiger nichtionischer Tensidkomponenten wird das sofort ersichtlich:

Naturstoffbasierte Komponenten der hier genannten Art haben als oleophilen Bestandteil in der Regel Fettalkohole beziehungsweise Fettalkoholgemische natürlichen Ursprungs. Diese Fettalkohole werden dabei ihrerseits durch Reduktion der aus natürlichen Ölen und Fetten gewonnenen Carbonsäuren gewonnen. Diese intermediär hergestellten Fettalkohole werden dann mit hydrophil wirkenden Funktionselementen versehen. Im Falle der Aniontenside wird beispielsweise eine Sulfonierung und/oder eine Sulfatierung, in der Regel unter Einsatz von SO₃ oder Chlorsulfonsäure, durchgeführt. Zur Herstellung von Fettalkohol-basierten niotensidischen Komponenten werden wasserlösliche Reste durch Alkoxylierung oder durch Umsatz mit wasserlöslichen Glykoseresten eingeführt. Alle Reaktionen finden unter verschärften Verfahrensbedingungen, insbesondere erhöhten Temperaturen, statt. Dabei sind die Einsatzmaterialien natürlichen Ursprungs in aller Regel keine definierten Verbindungen, sondern Stoffgemische, so wie sie die Natur zur Verfügung stellt und wie sie aus solchen Stoffmischungen durch einfache Trennverfahren gewonnen werden können.

Es ist bekannt, daß sich aus diesen Voraussetzungen beträchtliche Anforderungen an die Reinigung der Einsatzmaterialien, der Reaktionszwischenprodukte und der gewünschten Endprodukte ableiten. Die Kostenstruktur von Wertstoffen und Wertstoffgemischen der hier betroffenen Art wird zu substantiellem Anteil durch den technischen Aufwand bestimmt, der für die jeweilige Reinigungsstufe einzusetzen ist.

Die DE-A-34 47 867 beschreibt ein Verfahren zur Reinigung von konzentrierten wäßrigen Tensidgemischen auf Basis von Alkyl- und/oder Alkarylpolyglycolethersulfatsalzen, inbesondere zur Abtrennung von 1,4-Dioxan aus diesen Tensidgemischen, das dadurch gekennzeichnet ist, daß man das wäßrige Tensidgemisch nach Zusatz geringer Mengen eines Entschäumungsmittels auf Basis ethoxylierter Silikonöle mit einem auf Temperaturen über 100°C erhitzten Wasserdampfstrom unter intensiver Verwirbelung der Flüssig- und der Dampfphase durchströmt. Die DE-A-26 22 520 beschreibt ein Verfahren zur kontinuierlichen Vorreinigung und Bleichung von Speiseölen und ähnlichen Substanzen sowie eine Anlage zur Durchführung dieses Verfahrens. Das Verfahren kennzeichnet sich dadurch, daß das zu behandelnde Produkt nacheinander durch mehrere liegende Zylinder geführt wird, wobei im ersten Zylinder eine Reaktion mit dosiert zugeführten Reagenzstoffen erfolgt, im 2. Zylinder eine Vakuumtrocknung bei gleichzeitiger Verwirbelung der Mischung mittels überhitzten Dampfes und Wärmezufuhr erfolgt und anschließend nach Zuführung eines Bleichmittels die Mischung in den 3. Zylinder für eine ebenfalls wählbare Verweilzeit geführt wird.

Wertstoffe und Wertstoffgemische für den Einsatz in Netz-, Wasch-und/oder Reinigungsmitteln können als Flüssigkomponenten oder in Form eines schüttfähigen kornförmigen Gutes vorliegen. Sie werden in aller Regel durch Trocknungsverfahren intermediär erhaltener wäßriger Zubereitungen dieser Stoffklasse gewonnen. Die Sprühtrocknung entsprechender wäßriger Zubereitungen findet seit Jahrzehnten weltweit in großtechnischem Maßstab statt. Als Trocknungsgasstrom werden Heißluft beziehungsweise Gemische von Luft und heißen Verbrennungsgasen eingesetzt.

Umfangreiche Arbeiten der Anmelderin beschäftigen sich mit der Einsatzmöglichkeit des Arbeitsprinzips der Heißdampftrocknung auf Wertstoffe und Wertstoffgemische aus dem Bereich der Netz-, Wasch-und/oder Reinigungsmittel. Es wurde überraschenderweise gefunden, daß die insbesondere ökologischen und energetischen Vorteile der Heißdampftrocknung auch für dieses an sich sehr sensitive Arbeitsbeziehungsweise Produktgebiet genutzt werden können. So beschreibt die DE-A 40 30 688 ein Verfahren zur Gewinnung feinteiliger fester schütt- beziehungsweise rieselfähiger Wertstoffe oder Wertstoffgemische für Netz-, Wasch- und/oder Reinigungsmittel aus ihren wäßrigen Zubereitungen, wobei überhitzter Wasserdampf als trocknender Heißgasstrom eingesetzt wird. Dabei wird die Trocknung des partikulären Gutes vor dessen Gefährdung durch thermische Einwirkung abgebrochen.

In einer Reihe weiterer älterer Anmeldungen der Anmelderin werden besondere Ausgestaltungen und Verbesserungen solcher Trocknungsverfahren mit überhitztem Wasserdampf als Heißgasstrom und deren Anwendung auf das Gebiet der Wertstoffe und Wertstoffgemische für Netz-, Wasch- und/oder Reinigungsmittel geschildert. Verwiesen wird in diesem Zusammenhang insbesondere auf die älteren deutschen Patentanmeldungen DE-P 42 04 035.3, DE-P 42 04 090.6, DE-P 42 06 050.8, DE-P 42 06 521.6, DE-P 42 06 495.3, DE-P 42 08 773.2 und DE-P 42 09 432.1.

Eine besonders wichtige weiterführende Entwicklung beschäftigt sich mit den unerwarteten vorteilhaften Produkteigenschaften, die ein durch Heißdampftrocknung erhaltenes festes schütt- und rieselfähiges Einsatzgut aufweist. Beschrieben wird hier, wie in einem primär durch Heißdampftrocknung anfallenden Einsatzgut eine mikroporöse Gutstruktur ausgebildet und fixiert werden kann und wie darauf aufbauend Wertstoffabmischungen des hier betroffenen Produktbereiches in bisher nicht bekannter Weise ausgestaltet werden können.

Verwiesen wird in diesem Zusammenhang auf die ältere deutsche Patentanmeldung P 42 34 376.3 der Anmelderin.

Die im nachfolgenden gegebene erfindungsgemäße Lehre zu Gewinnung von Wertstoffen und Wertstoffgemischen der angegebenen Art mit erhöhter Reinheit bei gleichzeitig deutlich vermindertem Arbeitsaufwand baut auf den Erkenntnissen und Arbeitsregeln aus der genannten DE-A-40 30 688 und den genannten älteren Anmeldungen auf. Zum Zwecke der Erfindungsoffenbarung wird dementsprechend hiermit ausdrücklich der Offenbarungsinhalt dieser Veröffentlichung und der genannten älteren Anmeldungen auch zum Gegenstand der vorliegenden Erfindungsoffenbarung gemacht, der in Kombination mit den nachfolgend angegebenen weiterführenden Erkenntnissen und Arbeitsregeln zu verstehen ist.

Die erfindungsgemäße Lehre geht von der überraschenden Erkenntnis aus, daß die Behandlung eines im Rahmen der Erfindungsbeschreibung offenbarten Einsatzgutes unter den Bedingungen der Behandlung mit überhitztem Wasserdampf nicht etwa nur zur Auftrocknung führt, sondern daß diese Arbeitsweise mit besonderem Vorteil zur Reinigung der Wertstoffe und Wertstoffgemische von unerwünschten Bestandteilen eingesetzt werden kann. Hierbei ist es möglich, kleinere aber auch gegebenenfalls größere Mengen an Fremdstoffen durch eine vergleichsweise kurzfristige Behandlung im erfindungsgemäßen Sinne aus dem zu reinigenden Einsatzgut zu entfernen. Dabei werden nicht nur beispielsweise aus der geruchssensorischen Wahrnehmung störende Komponenten aus dem Einsatzgut entfernbar, die Lehre der Erfindung schließt - soweit erforderlich - eine gleichzeitig bleichende Behandlung des Einsatzgemisches in ihr technisches Handeln ein. Aus diesen Möglichkeiten leitet sich zur Herstellung hochwertiger Wertstoffe und Wertstoffgemische der angegebenen Art ein vereinfachtes Reinigungsverfahren ab, durch das die Reinheitsanforderungen einfacher und damit kostengünstiger erfüllbar sind.

### Beschreibung der Erfindung

Gegenstand der Erfindung ist dementsprechend in einer ersten Ausführungsform ein Verfahren zur Verbesserung des Reinheitsgrades und insbesondere der Beschaffenheit von Farbe und Geruch in Wertstoffen und Wertstoffgemischen aus dem Bereich der Netz-, Wasch- und/oder Reinigungsmittel - im nachfolgenden als "Einsatzgut" bezeichnet - die auch in wäßriger Zubereitungsform vorliegen können, durch deren Behandlung mit einem Strom überhitzten Wasserdampfes bei ggf. gleichzeitiger Auftrocknung eines wasserhaltigen Einsatzgutes mittels Heißdampftrocknung. Das Verfahren ist dadurch gekennzeichnet, daß man ein mit Verunreinigungen belastetes Einsatzgut in feinverteilter Form und im Bereich des Normaldrucks in den Strom des überhitzten Wasserdampfs einträgt, gewünschtenfalls zur Ausbildung von Farbverbesserungen Bleichmittel mitverwendet und dabei diese Behandlung des verunreinigten Einsatzgutes in einer von dem überhitzten Wasserdampf durchströmten Sprühzone und/oder Wirbelschicht vornimmt.

Die erfindungsgemäße Lehre umfaßt die Anwendung des Verfahrens zur Trocknung wäßriger Zubereitungen von Wertstoffen und/oder Wertstoffgemischen aus dem erfindungsgemäß definierten Bereich in der Sprühzone und/oder in der Wirbelschicht, unter Einsatz von überhitztem Wasserdampf als Trocknungsgas zur gleichzeitigen Reinigung eines mit insbesondere wasserdampfflüchtigen Verunreinigungen beladenen Einsatzgutes.

Die Erfindung macht damit in wichtigen Ausführungsformen davon Gebrauch, daß eine Vielzahl von unerwünschten Begleitstoffen und Verunreinigungen des hier betroffenen Arbeitsgebietes im Vergleich zu den letztlich im Wertstoff beziehungsweise Wertstoffgemisch angestrebten Fertigkomponenten eine vergleichsweise erhöhte Flüchtigkeit, insbesondere in einer wasserdampferfüllten Atmosphäre, aufweisen. Dieser Sachverhalt gilt nicht etwa nur für niedermolekulare, gleichwohl schwer zu entfernende Reststoffe wie Ethylenoxid und/oder Propylenoxid sowie Dioxan aus entsprechenden Reaktionsgemischen, auch unerwünschte Begleitstoffe höheren Molgewichtes wie nicht-reagierte Anteile eingesetzter Fettalkohole beziehungsweise Fettalkoholgemische oder sich daraus ableitende partielle Oxidationsprodukte, insbesondere niedere Carbonylverbindungen wie Fettaldehyde, lassen sich in einer inerten Wasserdampfatmosphäre wirkungsvoll aus ihren Abmischungen mit den angestrebten Reinstoffen beziehungsweise Reingemischen des Einsatzgutes entfernen.

Reinigungsstufen bei der Herstellung hier betroffener Wertstoffe und Wertstoffgemische unter Einsatz von Wasserdampf sind an sich bekannt. Bekannt ist auch die gegebenenfalls wirkungsvolle Unterstützung, die die Mitverwendung von Wasserdampf bei der Entfernung von störenden Fremdstoffen in größerer oder kleinerer Menge leisten kann. So ist beispielsweise die Entfernung nicht umgesetzter Fettalkoholanteile im Rahmen der Herstellung von niotensidischen Komponenten aus der Klasse der Alkylpolyglykoside (APG) beschrieben in EP-B-0 092 876 (Procter & Gamble). Ganz allgemein ist aber auch die Reinigung von naturstoffbasierten Wertstoffen oder Wertstoffgemischen durch das sogenannte Dämpfen eine bekannte Möglichkeit, insbesondere störende Spurenstoffe aus dem zu reinigenden Gut zu entfernen (vgl. EP-B-0 283 862, Henkel)

Die Erfindung geht über diese Vorschläge des Standes der Technik hinaus. Die Behandlung des zu reinigenden Einsatzgutes erfolgt unter der Einwirkung des überhitzten Wasserdampfes, wie er als Trocknungsgas in der zitierten DE-A-40 30 688 für Wertstoffe und Wertstoffgemische der hier betroffenen Art erstmals beschrieben wird. Die dort offenbarte Trocknung kann im Rahmen des erfindungsgemäßen Handelns mitverwendet werden. Die Lehre der Erfindung geht aber darüber hinaus: Die Erfindung macht es jetzt erstmalig möglich ein mit Verunreinigungen belastetes Einsatzgut einem solchen Trocknungsverfahren zu unterwerfen und gleichwohl die angestrebten hochgereinigten Endprodukte zu erhalten. Es leuchtet ein, daß hierdurch wesentliche Vereinfachungen im Rahmen der mehrstufig ausgelegten Gewinnung von Wertstoffen der erfindungsgemäß betroffenen Art möglich wird. In Anpassung an die jeweiligen Gegebenheiten können substantielle Einsparungen bei der intermediären Reinigung von Vorstufen oder Endprodukten vor ihrer letztlichen Auftrocknung zum gewünschten Fertigprodukt vorgenommen werden. Gegenüber der konventionellen Trocknung in der Sprühzone und/oder der Wirbelschicht mit einer Heißgasphase auf Basis von Luft und Brenngasen eröffnen sich damit neue Möglichkeiten zur Gewinnung höchstwertiger Wertstoffe und Wertstoffgemische der betroffenen Art.

Besondere Bedeutung hat die Lehre der Erfindung im Zusammenhang mit der Gewinnung von Produkten, die frei von unerwünschten Geruchsbelastungen sind. Unabhängig oder gleichzeitig ermöglicht die erfindungsgemäße Lehre aber auch die Aufbesserung der Farbe der Endprodukte in Richtung auf hellfarbige Produkte eines hohen Weißgrades.

Die Entfernung der geruchlich sensorisch störenden Fremdkomponenten ist das unmittelbare Ergebnis der Behandlung des Gutes in der Phase des überhitzten Wasserdampfes. In der bevorzugten Ausführungsform der Erfindung werden dabei wasserhaltige Einsatzgutgemische in feinverteilter Form in eine mit überhitztem Wasserdampf durchströmte Zone eingetragen. Hier tritt dann der folgende Reaktionsablauf ein: Das in der Regel bei Temperaturen unterhalb der Verdampfungstemperatur des Wassers bei Arbeitsbedingungen eingebrachte wasserhaltige Flüssigkeitströpfchen führt zur sofortigen Kondensation weiteren Wassers auf dem Flüssigkeitströpfchen. Gleichzeitig geht die Kondensationswärme auf das tropfenförmige Gut über. Dieser Verdünnungsprozeß findet solange statt, bis die Temperatur des eingetragenen Gutes der Verdampfungstemperatur des Wassers unter Arbeitsbedingungen entspricht. In der Regel liegt diese Temperatur damit im Bereich um 100°C. Ist die Verdampfungstemperatur im Tropfen erreicht, so erfolgt in dem Einstoffsystem Wasser/Wasserdampf die Trocknung im gesamten Tropfen ohne Ausbildung einer starren Außenhülle, wie sie in konventionellem Trocknungsverfahren gebildet wird. Die Einzelheiten dieses Trocknungsverfahrens sind in der zitierten älteren deutschen Patentanmeldung P 42 34 376.3 im einzelnen geschildert, auf die hier nochmals ausdrücklich Bezug genommen wird. Das Ergebnis eines solchen Auftrocknungsprozesses im überhitzten Wasserdampf ist die in dieser älteren Anmeldung geschilderte hochporöse Innenstruktur des bei der Trocknungstemperatur festen Trockenguts. Dieser hochporöse Materialaufbau wird im Sinne der erfindungsgemäßen Lehre sinnvoll genutzt. Die im Prinzip flüchtigen und insbesondere wasserdampfflüchtigen Fremdstoffe werden nicht im Tropfeninneren eingeschlossen und am Abdampfen behindert, sie können während des Trocknungsprozesses - oder auch noch danach - unter der Einwirkung des überhitzten Dampfes in die Gasphase übergehen und werden mit dem überhitzten Wasserdampf ausgetragen. Zurück bleibt das gereinigte Gut. Eine entsprechende Erleichterung des Austrages von Fremdstoffkomponenten ist bei der Behandlung von Flüssigkomponenten in der überhitzten Dampfphase sichergestellt.

Die hier diskutierte Möglichkeit der Befreiung von Fremdstoffen wirkt sich vornehmlich auf die Beseitigung unerwünschter geruchsprägender Komponenten und auf die Entfernung geruchsneutraler vergleichsweise leichter flüchtiger beziehungsweise Heißdampftransportierbarer Komponenten aus. Die Lehre der Erfindung reicht insoweit darüber hinaus, als auch die gleichzeitige Beseitigung farblicher Verunreinigungen aus einem entsprechend belasteten Einsatzgut möglich wird. Hier sieht die erfindungsgemäße Lehre vor, Bleichmittel im Einsatzgut mit zu verwenden. Als Bleichmittel kommen sowohl oxidativ als auch reduktiv wirkende an sich bekannte Zusatzstoffe in Betracht. Typische Beispiele für oxidativ wirksame Komponenten sind Wasserstoffperoxid, Persalze wie Percarbonate, Perborate, Persulfate, Hypochloritsalze, insbesondere Alkalihypochlorit, und dergleichen. In an sich bekannter Weise können zusammen mit diesen oxidativ wirkenden Bleichmitteln sogenannte Bleichaktivatoren mitverwendet werden, wie sie aus dem Gebiet der Wasch- und Textil- hilfsmittel bekannt und im nachfolgenden noch im einzelnen beschrieben sind.

Beispiele für reduktiv wirkende Bleichkomponenten sind insbesondere solche Verbindungen dieser Stoffklasse, die in Gegenwart des überhitzten Wasserdampfes nicht zu unerwünschten Sekundärreaktionen führen. Geeignet sind beispielsweise Phosphorverbindungen, insbesondere unterphosphorige Säure und ihre Salze, insbesondere Alkali-und/oder Erdalkalisalze.

Bleichmittel können den zu reinigenden Einsatzgemischen in der jeweils benötigten Menge zugesetzt werden. Üblicherweise reichen selbst bei vergleichsweise starken Verunreinigungen Mengen bis etwa 10 Gew.-% aus, vorzugsweise werden Mengen im Bereich von etwa 0,5 bis 5 Gew.-% dem zu bleichenden Einsatzgut zugesetzt - Gew.-% bezogen auf die jeweils zu bleichende Komponente beziehungsweise das entsprechende Mehrkomponentengemisch.

Die Notwendigkeit zur Bleiche und/oder das Bedürfnis zur zusätzlichen Aufhellung wird entsprechende Maßnahmen des erfindungsgemäßen Handelns wünschenswert machen, wenn Einzelkomponenten mehr oder weniger starke Verfärbungen - üblicherweise insbesondere Bräunungen aus einzelnen Synthesestufen - aufweisen und/oder wenn insgesamt eine zusätzliche Aufhellung des Mehrkomponentengemisches, beispielsweise eines Textilwaschmittels oder eines entsprechenden Turmpulvers zur Herstellung von Textilwaschmitteln, gewünscht wird. Entsprechende Verfärbungen bei Einzelkomponenten sind bekannte Störfaktoren bei der Sulfonierung und/oder Sulfatierung oleophiler Komponenten im Rahmen der Herstellung von tensidisch wirkenden Verbindungen vom Aniontensidtyp. Vergleichsweise können aber auch entsprechende farbliche Probleme bei der Herstellung von quartäre Ammoniumgruppen-enthaltenden Komponenten mit Tensidcharakter vorliegen, wie sie beispielsweise im Rahmen von Weichspülern zur abschließenden Textilausrüstung nach der Wäsche Verwendung finden. Ein anderes bekanntes Beispiel für das Auftreten unerwünschter Bräunungen ist in der Klasse der niotensidischen APG-Verbindungen gegeben. Insbesondere der Glucosegehalt dieser Stoffklasse führt aufgrund seiner Temperaturempfindlichkeit leicht zu nicht akzeptablen Verbräunungen. Die erfindungsgemäße Lehre gibt hier eine besondere Arbeitshilfe zur Farbaufhellung. Durch gezielte Temperatursteuerung der überhitzten Wasserdampfphase kann mit Sicherheit der Grenztemperaturbereich vermieden werden, ab dem Bräunungen in dem APG-haltigen Gut üblicherweise auftreten. Zur gleichen Zeit ist in der erfindungsgemäß bevorzugten Form der Aufarbeitung über ein feinteiliges Einsatzgut in der Gasphase des überhitzten Wasserdampfes die Gewähr gegeben, daß keine Anbackungen und damit punktuelle Überhitzungen im APG-haltigen Gut an Metallflächen auftreten. Ein weiteres bekanntes Beispiel für problematische Farbgebungen im Tensid sind die Aniontenside auf Basis der alpha-substituierten Estersulfonate, hier insbesondere die entsprechenden Methylestersulfonatsalze (MES), sowie die sich daraus durch Esterspaltung ableitenden Di-Salze der alpha-Sulfofettsäuren.

Eine besondere Ausführungsform der Erfindung sieht vor, die Behandlung des zu reinigenden Einsatzgutes mit einer überhitzten Wasserdampfphase durchzuführen, der Spurenmengen an Ozon zugesetzt worden sind. Dieser Ozonzusatz kann insbesondere im Zusammenhang mit der Aufbesserung des geruchlich sensorischen Eigenschaftsbildes des zu behandelnden Einsatzgutes wichtig sein. Dabei können bereits sehr geringe Mengen an Ozon im Heißdampf zu einer wahrnehmbaren geruchlichen Aufbesserung des Gutes führen. Es ist in diesem Sinne erfindungsgemäß bevorzugt, Ozonzusätze im unteren ppm-Bereich vorzusehen, wobei die Bezugsgröße zu dieser Mengenangabe die jeweils aus dem Trocknungskreislauf des Heißdampf ausgeschleuste Brüdenteilstrommenge ist, die dem im Rahmen des Verfahrens verdampften und mit dem zu reinigenden Einsatzgut zugeführten Wasser entspricht. Bevorzugt liegt der Anteil an zugesetztem Ozon bei Werten nicht über etwa 1 ppm und insbesondere bei Mengen unterhalb 0,5 ppm. Ein besonders geeigneter Bereich für den Ozonzusatz kann im Bereich von etwa 0,05 bis 0,2 ppm liegen. Als Bemessungsgrundlage für diese ppm-Zahlenwerte gelten die zuvor gemachten Angaben. Der Zusatz des Ozons kann in den Kreislaufdampf - hier bevorzugt unmittelbar vor der Rückführung des Heißdampfes in die Trocknungszone - und/oder unmittelbar in die Trocknungszone erfolgen.

In einer bevorzugten Ausführungsform wird ein verunreinigtes Einsatzgut der erfindungsgemäßen Behandlung unterworfen, das eine wenigstens kurzfristige Temperaturerhöhung auf die Verdampfungstemperatur des Wassers unter Verfahrensbedingungen, insbesondere auf Temperaturen im Bereich von etwa 90 bis 110°C, wenigstens weitgehend schadlos übersteht. Wenn auch hier flüssige und/oder feste Einsatzmaterialien gleicherweise geeignet sind, so lassen sich häufig besonders gute Ergebnisse bei Materialien erhalten, die im Temperaturbereich von 90 bis 110°C im aufgetrockneten Zustand als Feststoff vorliegen und als zu reinigendes Einsatzmaterial insbesondere geruchlich und/oder farblich nicht befriedigen.

Es ist weiterhin bevorzugt, fließfähige und versprühbare wäßrige Lösungen, Emulsionen und/oder Suspensionen des verunreinigten Einsatzgutes der Behandlung mit dem überhitzten Wasserdampf zu unterwerfen. Hierbei kann in der Sprühzone und/oder in der Wirbelschicht eine wenigstens anteilsweise Trocknung der wasserhaltigen fließfähigen Materialien mit der erfindungsgemäßen Veredelungsstufe verbunden werden.

Die Parameter zur Behandlung des Einsatzgutes mit dem überhitzten Wasserdampf entsprechen im übrigen den Angaben der DE-A 40 30 688 und den in diesem Zusammenhang genannten älteren Anmeldungen der Anmelderin. Zur Zusammensetzung geeigneter Wertstoffe und Wertstoffgemische für Netz-, Wasch- und/oder Reinigungsmittel wird insbesondere auf die ältere deutsche Patentanmeldung P 42 34 376.3 verwiesen. Hier werden im Temperaturbereich von 100 bis 110°C und bevorzugt im Temperaturbereich bis etwa 120°C als Feststoff vorliegende Wertstoffe beziehungsweise Wertstoffgemische des hier betroffenen Arbeitsgebietes beschrieben, deren Plastizität und Oberflächenklebrigkeit derart eingeschränkt sind, daß substantielle Verklebungen der Teilchen miteinander und/oder Verklebungen der offenporigen Teilcheninnenstruktur auch unter den Bedingungen der Einwirkung des überhitzten Wasserdampfes ausscheiden. Die erfindungsgemäße Lehre macht von diesen Elementen der Lehre der älteren Anmeldung in wichtigen Ausführungsformen Gebrauch. Dabei können sowohl einzelne Wertstoffe wie Trägerbeads im Sinne dieser älteren Anmeldung aus Wertstoffgemischen als auch schließlich die zur Beladung dieser Trägerbeads eingesetzten Wertstoffe einer Reinigung im Sinne der erfindungsgemäßen Lehre unterworfen werden beziehungsweise unterworfen worden sein.

Zur Durchführung des erfindungsgemäßen Reinigungsverfahrens wird im Bereich des Normaldrucks oder bei nur schwach erhöhten oder nur schwach erniedrigten Drucken gearbeitet. So sind bevorzugte Abweichungen vom Normaldruck nach beiden Seiten Druckabweichungen bis zu etwa 0,15 bar, vorzugsweise nicht mehr als etwa 0,1 bar und insbesondere nicht mehr als 0,01 bis 0,05 bar. Das Arbeiten bei schwach erhöhten Innendrucken des Systems schließt auch beim Auftreten von Schadstellen einen unerwünschten Einbruch von sauerstoffhaltiger Außenluft und damit unerwünschte Sekundärreaktionen mit dem zu behandelnden Wertstoffgut aus. Die Einsatztemperaturen des überhitzten Wasserdampfes liegen üblicherweise im Bereich von 100 bis 450°C und vorzugsweise im Bereich von etwa 115 bis 350°C. Im übrigen wird in diesem Zusammenhang auf die Angaben der genannten Schutzrechtsserie der Anmelderin zu dem hier betroffenen Arbeitsgebiet verwiesen. Die im Arbeiten mit überhitztem Wasserdampf erforderlichen geschlossenen Kreislaufströme bringen für das erfindungsgemäße Arbeiten einen wesentlichen Vorteil: Der mit den ausgetragenen Verunreinigungen beladene Wasserdampfstrom beziehungsweise -teilstrom wird nicht als solcher in die Atmosphäre entlassen, sondern der Kondensation unterworfen. Der überwiegende Anteil ausgetragener Verunreinigungen wird bei einer solchen Kondensation mit in die flüssige und/oder feste Phase übergehen. In der Gasphase verbleibende Restanteile können zusammen mit der aus dieser nachfolgenden Kondensationsstufe abgetrennten Gasphase in geeigneter Weise aufgearbeitet, beispielsweise der Verbrennung zugeführt werden. Die bei der Kondensation des ausgekreisten Brüdenteilstromes angefallenen ausgetragenen Anteile an ehemaligen Ausgangsstoffen des zur reinigenden Einsatzgutes können - je nach ihrer Beschaffenheit - schadfrei vernichtet werden oder in geeignete Vorstufen der Gewinnung der jeweiligen Wertstoffe zurückgeführt werden. Es gelingt in dieser Weise die völlig sichere, von Umweltschädigungen freie und wirtschaftlich optimierte Reinigung der jeweiligen Einsatzmaterialien auch dann, wenn bei der Herstellung des zu reinigenden Wertstoffes bis heute übliche kostspielige Teilschritte - insbesondere destillative Reinigungen und dergleichen - nicht oder nicht in der bis heute üblichen Intensität vorgenommen worden sind.

Im nachfolgenden werden ohne Anspruch auf Vollständigkeit weiterführende Angaben zu Wert- und Hilfsstoffen des hier betroffenen Sachgebietes gemacht, wobei ausdrücklich hiermit auf das darüberhinausgehende allgemeine Wissen der Fachwelt verwiesen wird.
a1) Als anionische Tenside mit Sulfat- oder Sulfonatstruktur eignen sich beispielsweise Alkylbenzolsulfonate, Alkansulfonate, Olefinsulfonate, Alkylethersulfonate, Glycerinethersulfonate, alpha-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Sulfosuccinate, Sulfosuccinamate, Sulfotriglyceride, Isethionate, Tauride und Alkyloligoglucosidsulfate. Sofern die anionischen Tenside Polyglycoletherketten enthalten, können sie eine konventionelle, vorzugsweise jedoch eine eingeengte Homologenverteilung aufweisen.
a2) Als anionische Tenside mit Carboxylatstruktur kommen zum Beispiel Seifen aus natürlichen oder synthetischen, vorzugsweise gesättigten Fettsäuren in Betracht. Geeignet sind insbesondere aus natürlichen Fettsäuren, zum Beispiel Kokos-, Palmkern- oder Talgfettsäuren abgeleitete Seifengemische. Bevorzugt sind solche, die zu 50 bis 100% aus gesättigten C₁₂₋₁₈-Fettsäureseifen und zu 0 bis 50% aus Ölsäureseifen zusammengesetzt sind.
   Daneben eignen sich ferner auch Amidseifen, Ethercarbonsäure-Salze und Sarcosinate.
a3) Im Sinne der vorliegenden Erfindung sind unter dem Begriff "anionische Tenside mit Sulfat-, Sulfonat- und/oder Carboxylatstruktur" auch solche amphoteren beziehungsweise zwitterionischen Tenside zu verstehen, die mindestens eine dieser Gruppen im Molekül enthalten. Typische Beispiele sind Alkylbetaine, Alkylamidobetaine, Aminopropionate, Aminoglycinate, Imidazoliniumbetaine und Sulfobetaine.
   Die anionischen Tenside können in Form ihrer Natrium-, Kalium-und Ammoniumsalze sowie als lösliche Salze organischer Basen, wie Mono-, Di- oder Triethanolamin vorliegen. Bei den genannten Stoffen handelt es sich ausschließlich um bekannte Verbindungen. Hinsichtlich ihrer Struktur und Herstellung sei auf einschlägige Übersichtsarbeiten beispielsweise J. Falbe (ed.), "Surfactants in Consumer Products", Springer Verlag, Berlin, 1987, S. 54-124 oder J. Falbe (ed.), "Katalysatoren, Tenside und Mineralöladditive", Thieme Verlag, Stuttgart, 1978, S. 123-217, verwiesen.
b) Nichtionische Tensidverbindungen im Sinne der erfindungsgemäßen Lehre können als Wertstoffe die verschiedensten Aufgaben wahrnehmen. Einerseits sind sie im klassischen Sinne synergistisch mit den Aniontensiden zusammenwirkende waschaktive Komponenten. Zum anderen kann der Klasse von Niotensiden insbesondere aber auch weiterführende Bedeutung, beispielsweise im Sinne von Dispergatoren, Strukturbrechern und dergleichen, zukommen.
   Niontensidverbindungen können in an sich bekannter Weise bei Raumtemperatur flüssig oder fest sein. Unter Verweisung auf allgemeines Fachwissen seien hier aufgezählt:
b1) Die als nichtionische Tenside eingesetzten flüssigen Alkoholethoxylate leiten sich beispielsweise von primären Alkoholen mit vorzugsweise 9 bis 18 Kohlenstoffatomen und durchschnittlich 1 bis 12 Mol Ethylenoxid ab, in denen der Alkoholrest linear oder verzweigt, insbesondere in 2-Stellung methylverzweigt sein kann, beziehungsweise lineare und methylverzweigte Reste im Gemisch enthalten kann, so wie sie üblicherweise in Oxoalkoholresten vorliegen. Insbesondere sind jedoch lineare Reste aus Alkoholen nativen Ursprungs mit 12 bis 18 Kohlenstoffatomen bevorzugt, wie z.B. aus Kokos-, Talgfett- oder Oleylalkohol.
   Insbesondere bevorzugt können sein C₁₂₋₁₄-Alkohole mit 3 EO oder 4 EO, C₉₋₁₁-Alkohole mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO.
   Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Insbesondere sind Alkoholethoxylate bevorzugt, die durchschnittlich 2 bis 8 Ethylenoxidgruppen aufweisen.
b2) Als weitere nichtionische Tenside können auch Alkyloligoglykoside der allgemeinen Formel (I) eingesetzt werden,

   R¹-O-(G)ₓ (I)

   in der R¹ einen primären geradkettigen oder in 2-Stellung methylverzweigten aliphatischen Rest mit 8 bis 22, vorzugsweise 12 bis 18 C-Atomen bedeutet und G das Symbol ist, das für eine Glykoseeinheit mit 5 oder 6 E-Atomen, vorzugsweise für Glucose, steht. Der Oligomerisierungsgrad x, der die Verteilung von Monglykosiden und Oligoglykosiden angibt, ist eine beliebige Zahl zwischen 1 und 10 und liegt beispielsweise im Bereich von etwa 1,2 bis 4, insbesondere bei 1,2 bis 2.
b3) Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.
b4) Weitere geeignete Tenside sind Polyhydroxyfettsäureaminde der Formel (II), 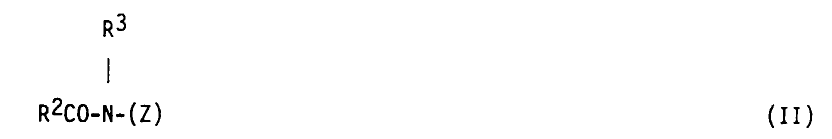 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und (Z) für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften US 1,985,424, 2,016,962 und 2,703,798 sowie die internationale Patentanmeldung WO 92/06984 (Procter & Gamble) verwiesen. Vorzugsweise leiten sich die Polyhydroxyfettsäureamine von reduzierenden Zuckern mit 5 oder 6 Kohlenstoffatomen, insbesondere von der Glucose ab. Die bevorzugten Polyhydroxyfettsäureaminde stellen daher Fettsäure-N-alkylglucamide dar, wie sie durch die Formel (III) wiedergegeben werden: 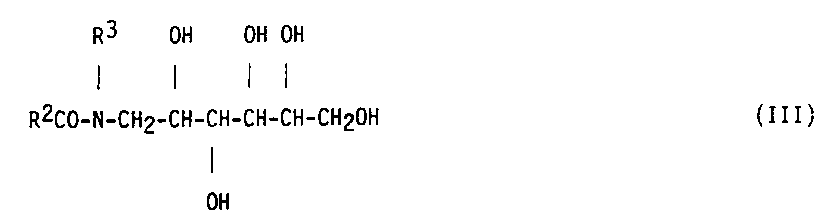 Vorzugsweise werden als Polyhydroxyfettsäureamide Fettsäure-N-alkyl-glucamide der Formel (III) eingesetzt, in der R³ für Wasserstoff oder eine Amingruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure beziehungsweise derer technischer Mischungen steht. Besonders bevorzugt sind Fettsäure-N-alkylglucamide der Formel (III), die durch reduktive Aminierung von Glucose mit Methylamin und anschließende Acylierung mit Laurinsäure oder C₁₂₋₁₄-Kokosfettsäure beziehungsweise einem entsprechenden Derivat erhalten werden.
c) Als organische und anorganische Builder- beziehungsweise Gerüstsubstanzen - die im Rahmen erfindungsgemäß zu reinigender Wertstoffgemische mitverwendet werden können - eignen sich schwach sauer, neutral oder alkalisch reagierende lösliche und/oder unlösliche Komponenten, die Calciumionen auszufällen oder komplex zu binden vermögen.
c1) Geeignete und insbesondere ökologisch unbedenkliche Buildersubstanzen, wie feinkristalline, synthetische wasserhaltige Zeolithe vom Typ NaA, die ein Calciumbindevermögen im Bereich von 100 bis 200 mg CaO/g (gemäß den Angaben in DE-A 24 12 837 aufweisen, finden eine bevorzugte Verwendung. Ihre mittlere Teilchengröße liegt üblicherweise im Bereich von 1 bis 10 µm (Meßmethode: Coulter Counter, Volumenverteilung). Geeignete Substitute beziehungsweise Teilsubstitute für Phosphate und Zeolithe sind kristalline, schichtförmige Natriumsilicate der allgemeinen Formel (IV),

   NaMSiₓO₂ₓ₊₁ ∗y H₂O (IV)

   wobei M Natrium oder Wasserstoff bedeutet, x eine Zahl von 1,9 bis 4 und y eine Zahl von 0 bis 20 ist und bevorzugte Werte für x 2, 3 oder 4 sind. Derartige kristalline Schichtsilikate werden beispielsweise in der europäischen Patentanmeldung EP-A 0 164 514 beschrieben. Bevorzugte kristalline Schichtsilikate der Formel (IV) sind solche, in denen M für Natrium steht und x die Werte 2 oder 3 annimmt. Insbesondere sind sowohl beta- als auch delta-Natriumdisilicate der Formel (V)

   Na₂Si₂O₅ ∗y H₂O (V)

   bevorzugt, wobei beta-Natriumdisilikat beispielsweise nach dem Verfahren erhalten werden kann, das in der deutschen Patentanmeldung DE-A 39 39 919 beschrieben ist.
c2) Als weitere Builderbestandteile, die insbesondere zusammen mit den Zeolithen eingesetzt werden können, kommen Schichtverbindungen vom Hydrotalcit-Typ sowie (co-)polymere Polycarboxylate in Betracht, wie Polyacrylate, Polymethacrylate und insbesondere Copolymere der Acrylsäure mit Maleinsäure, vorzugsweise solche aus 50% bis 10% Maleinsäure. Die relative Molekülmasse der Homopolymeren liegt im allgemeinen zwischen 1.000 und 100.000, die der Copolymeren zwischen 2.000 und 200.000, vorzugsweise 50.000 bis 120.000, bezogen auf freie Säure. Ein besonders bevorzugtes Acrylsäure-Maleinsäure-Copolymer weist eine relative Molekülmasse von 50.000 bis 100.000 auf. Geeignete, wenn auch weniger bevorzugte Verbindungen dieser Klasse sind Copolymere der Acrylsäure oder Methacrylsäure mit Vinylethern, wie Vinylmethylethern, in denen der Anteil der Säure mindestens 50% beträgt. Brauchbar sind ferner Polyacetalcarbonsäuren, wie sie beispielsweise in den US-Patentschriften US 4,144,226 und US 4,146,495 beschrieben sind sowie polymere Säuren, die durch Polymerisation von Acrolein und anschließende Disproportionierung mittels Alkalien erhalten werden und aus Acrylsäureeinheiten und Vinylalkoholeinheiten beziehungsweise Acroleineinheiten aufgebaut sind.
c3) Brauchbare organische Gerüstsubstanzen sind ferner die bevorzugt in Form ihrer Natriumsalze eingesetzten Polycarbonsäuren, wie Citronensäure, Ethylendiamintetraessigsäure (EDTA) und Nitrilotriessigsäure (NTA), sofern ein derartiger Einsatz aus ökologischen Gründen nicht zu beanstanden ist.
c4) Weitere geeignete Inhaltsstoffe der Mittel sind wasserlösliche anorganische Alkalisierungsmittel wie Bicarbonate, Carbonate oder Silicate; insbesondere werden Alkalicarbonat und Alkalisilicat, vor allem Natriumsilicat mit einem molaren Verhältnis von Na₂O : SiO₂ von 1 : 1 bis 1 : 4,0, eingesetzt.
d) Zu den sonstigen Waschmittelbestandteilen zählen Vergrauungsinhibitoren (Schmutzträger), Schaumregulatoren, Bleichaktivatoren, optische Aufheller, textilweichmachende Stoffe, Farb- und Duftstoffe sowie Neutralsalze. Wichtige weitere Wasch- und Reinigungsmittelbestandteile sind beispielsweise Bleichmittel und Enzyme. Wie weit diese Komponenten der Reinigungsbehandlung im Sinne der Erfindung bedürfen ist von Fall zu Fall zu prüfen. In der Zusammenstellung von Wertstoffen beziehungsweise Wertstoffgemischen für das erfindungsgemäße Einsatzgebiet werden Komponenten dieser Art, üblicherweise getrennt, letztlich den zum Einsatz kommenden Wertstoffgemischen zugesetzt. Eine Ausnahme hiervon können die Bleichaktivatoren sein, die - wie zuvor angegeben - als Hilfsstoffe zur verbesserten Bleiche unter den erfindungsgemäßen Bedingungen auch in den Wertstoffgemischen unmittelbar während der Behandlung mit dem überhitzten Wasserdampf mitverwendet werden können. Gleichwohl wird auch in einem solchen Fall für ein fertiges Wertstoffgemisch, beispielsweise im Sinne eines Textilwaschmittels der nachträgliche Zusatz von zusätzlichen Mengen an Bleichaktivatoren zweckmäßig sein.
d1) Bleichaktivatoren die insbesondere für den Einsatz von Wasch-und Reinigungsmitteln bei Temperaturen von 60°C und darunter zu einer verbesserten Bleichwirkung führen sollen, sind beispielsweise mit H₂O₂ organische Persäuren bildende N-Acyl- beziehungsweise O-Acyl-Verbindungen, vorzugsweise N,N'-tetraacylierte Diamine, wie N,N,N',N'-Tetraacetylethylendiamin, ferner Carbonsäureanhydride und Ester von Polyolen wie Glucosepentaacetat.
d2) Vergrauungsinhibitoren haben die Aufgabe, den von der Faser abgelösten Schmutz in der Flotte suspendiert zu halten und so das Vergrauen zu verhindern. Hierzu sind wasserlösliche Kolloide meist organischer Natur geeignet, wie beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche saure Gruppen enthaltende Polyamide sind für diesen Zweck geeignet. Weiterhin lassen sich lösliche Stärkepräparate und andere als die oben genannten Stärkeprodukte verwenden, zum Beispiel abgebaute Stärke, Aldehydstärken usw. Auch Polyvinylpyrrolidon (PVP) ist brauchbar.
d3) Als Schaumregulatoren kommen Kombinationen geeigneter Tensidtypen in Betracht; eine Verringerung läßt sich ebenfalls durch Zusätze nichttensidartiger organischer Substanzen erreichen. Ein verringertes Schäumvermögen, das beim Arbeiten in Maschinen erwünscht ist, erreicht man vielfach durch Kombination verschiedener Tensidtypen, zum Beispiel von Sulfaten und/oder Sulfonaten mit nichtionischen Tensiden und/oder mit Seifen. Bei Seifen steigt die schaumdämpfende Wirkung mit dem Sättigungsgrad und der C-Zahl des Fettsäurerestes an. Als schauminhibierende Seifen eignen sich daher solche Seifen natürlicher und synthetischer Herkunft, die einen hohen Anteil an C₁₈₋₂₄-Fettsäuren aufweisen. Geeignete nichttensidartige Schauminhibitoren sind Organopolysiloxane und deren Gemische von mikrofeiner, gegebenenfalls silanierter Kieselsäure, Paraffine, Wachse, Mikrokristallinwachse und deren Gemische mit silanierter Kieselsäure. Auch von C₁₂₋₂₀-Alkylaminen und C₂₋₆-Dicarbonsäuren abgeleitete Bisacylamide sind brauchbar. Mit Vorteil werden auch Gemische verschiedener Schaumregulatoren verwendet, zum Beispiel solche aus Siliconen und Paraffinen oder Wachsen. Vorzugsweise sind die Schaumregulatoren beziehungsweise -inhibitoren an eine granulare in Wasser lösliche beziehungsweise dispergierbare Trägersubstanz gebunden.
d4) Als optische Aufheller können Derivate der Diaminostilbendisulfonsäure beziehungsweise deren Alkalimetallsalze vorliegen. Geeignet sind zum Beispiel als Salze 4,4'-Bis(2-aniliono-4-morpholino-1,3,5-triazin-6yl-amino)-stilben-2,2'disulfonsäure oder gleichartig aufgebaute Verbindungen, die anstelle der Morpholinogruppe eine Diethanolamingruppe, eine Methylaminogruppe, eine Anilinogruppe oder eine 2-Methoxyethylaminogruppe tragen. Weiterhin können Aufheller vom Typ des substituierten 4,4'-Distyryl-di-phenyls anwesend sein; zum Beispiel die Verbindung 4,4'-Bis(4-chlor-3-sulfostyryl)-diphenyl. Auch Gemische der vorgenannten Aufheller können verwendet werden.
d5) In an sich bekannter Weise können weitere Verbesserungen bezüglich des Weißgrades erhalten werden, wenn die Mittel außer den üblichen optischen Aufhellern auch geringe Mengen eines blauen Farbstoffes enthalten. Ein besonders bevorzugter Farbstoff ist Tinolux (R) (Produktname der Ciba-Geigy).
e) Zur Erhöhung der raschen Auflösung an sich schwerlöslicher Komponenten, zum Beispiel entsprechender Aniontenside wie FAS, auch bei Raumtemperatur oder nur mäßig erhöhten Temperaturen kann die Mitverwendung sogenannter Strukturbrecher wichtig sein, die häufig schon in sehr geringen Mengen - bezogen auf Aniontensid - hochwirksam sind. Eine Reinigung gerade auch solcher Strukturbrecher im Sinne des erfindungsgemäßen Verfahrens kann zu wichtigen Verbesserungen führen.
e1) Als Strukturbrecher eignen sich eine Reihe sowohl fester als auch flüssiger Substanzen, die hydrophil, wasserlöslich oder in Wasser dispergierbar sind. Geeignet sind beispielsweise niedere Polyalkylenglykole, die sich von geradkettigen oder verzweigtkettigen Glykolen mit 2 bis 6 Kohlenstoffatomen ableiten, vorzugsweise Polyethylenglycol oder Polypropylenglycol, und eine relative Molekülmasse zwischen 200 und 12.000 aufweisen. Insbesondere sind Polyethylenglycole mit einer relativen Molekülmasse zwischen 200 und 4.000 bevorzugt, wobei die flüssigen Polyethylenglycole mit einer relativen Molekülmasse bis 2.000 und insbesondere zwischen 200 und 600 besonders vorteilhafte Eigenschaften aufweisen.
e2) Ebenso sind die Sulfate und insbesondere die Disulfate von niederen Polyalkylenglycolen und zwar insbesondere des Polyethylenglycols und des 1,2-Propylenglycols geeignet. Besonders bevorzugt sind dabei die Sulfate und/oder Disulfate, die sich von Polyethylenglycolen und Polypropylenglycolen mit einer relativen Molekülmasse zwischen 600 und 6.000 und insbesondere zwischen 1.000 und 4.000 ableiten. Die Disulfate stammen dabei in der Regel von Polyglycolethern wie sie - bewirkt durch geringe Wasserspuren - bei der Oxalkylierung von alkoholischen Komponenten entstehen können, ab.
e3) Eine weitere Gruppe der geeigneten Strukturbrecher besteht aus den wasserlöslichen Salzen von Mono- und/oder Disulfosuccinaten der niederen Polyalkylenglycole. Besondere Bedeutung besitzen dabei wiederum die entsprechenden Polyethylenglycol- und Polypropylenglycol-Verbindungen, wobei Sulfosuccinate und Disulfosuccinate von Polyglycolethern mit einer relativen Molekülmasse zwischen 600 und 6.000, insbesondere zwischen 1.000 und 4.000 besonders bevorzugt sind.
   Für die Verwendung der anionisch modifizierten Polyalkylenglycole als Strukturbrecher kommen beliebige Salze, vorzugsweise jedoch die Alkalimetallsalze, insbesondere die Natrium- und Kalium-Salze, sowie Ammoniumsalze und/oder Salze von organischen Aminen, beispielsweise von Triethanolamin, in Betracht. Die für die praktische Anwendung wichtigsten Salze sind die Natriumsalze der Sulfate, Disulfate, Sulfosuccinate und Disulfosuccinate von Polyethylenglycol und Polpropylenglycol.
   Vorzugsweise werden auch Mischungen der Polyalkylenglycole und ihrer anionisch modifizierten Derivate in einem beliebigen Mischungsverhältnis eingesetzt. Insbesondere ist dabei eine Mischung aus Polyalkylenglycol und den Sulfosuccinaten und/oder Disulfosuccinaten der Polyalkylenglycole bevorzugt. Geeignet ist aber auch eine Mischung aus Polyalkylenglycol und den entsprechenden Sulfaten und/oder Disulfaten und eine Mischung aus Polyalkylenglycol und den entsprechenden Sulfaten und/oder Disulfaten sowie den entsprechenden Sulfosuccinaten und/oder Sulfodisuccinaten.
e4) Weiterhin sind im Sinne der Erfindung geeignete und bevorzugt eingesetzte Strukturbrecher die Anlagerungsprodukte von etwa 20 bis etwa 80 Mol Ethylenoxid an 1 Mol eines aliphatischen Alkohols mit im wesentlichen 8 bis 20 Kohlenstoffatomen, die seit langem bekannte Inhaltsstoffe von Wasch- und Reinigungsmitteln darstellen. Besonders wichtig sind die Anlagerungsprodukte von 20 bis 60 Mol und insbesondere 25 bis 45 Mol Ethylenoxid an primäre Alkohole, wie zum Beispiel Kokosfettalkohol oder Talgfettalkohol, an Oleylalkohol, an Oxoalkohole, oder an sekundäre Alkohole mit 8 bis 18 und vorzugsweise 12 bis 18 Kohlenstoffatomen. Beispiele für besonders bevorzugte Strukturbrecher aus der Gruppe der hochethoxylierten Alkohole sind Talgfettalkohol mit 30 EO und Talgfettalkohol mit 40 EO. Ebenso ist es bevorzugt, Mischungen einzusetzen, die hochethoxylierte Alkohole enthalten, beispielsweise Mischungen aus Talgfettalkohol mit 40 EO und Wasser oder aus Talgfettalkohol mit 40 EO und Polyethylenglycol mit einer relativen Molekülmasse zwischen 200 und 2.000.
e5) Weitere geeeignete Strukturbrecher sind ethoxylierte, vicinale innenständige Alkandiole oder 1,2-Alkandiole mit einer Kohlenstoffkette von 8 bis 18 Kohlenstoffatomen und 4 bis 15 Mol Ethylenoxid pro Mol Diol. Dabei ist es möglich, daß nur eine der beiden OH-Gruppen oder beide OH-Gruppen des Alkandiols ethoxyliert sind.
e6) Weiterhin sind als Strukturbrecher modifizierte nichtionische Tenside mit einer endständigen Säuregruppe geeignet. Hierbei handelt es sich um nichtionische Tenside, insbesondere um Fettalkohole, bei denen eine OH-Gruppe in eine Gruppe mit einer Carboxylgruppe umgewandelt wurde. Zu den nichtionischen Tensiden mit endständiger Säuregruppe gehören somit Ester oder Teilester eines nichtionischen Tensids mit einer Polycarbonsäure oder einem Polycarbonsäureanhydrid. Beispiele für säure-terminierte nichtionische Tenside sind die bekannten Polyethercarbonsäuren und Ester beziehungsweise Halbester von C₈₋₁₈-Alkoholen mit Bernsteinsäureanhydrid, Maleinsäureanhydrid, Maleinsäure oder Citronensäure.
e7) Eine weitere Gruppe geeigneter Strukturbrecher besteht aus Alkylenglycol-monoalkylethern der allgemeinen Formel (VI),

   R⁴O(CH₂CH₂O)ₙH (VI)

   in der R⁴ einen Rest mit 2 bis 6 Kohlenstoffatomen und n eine Zahl von 1 bis 8 darstellen. Beispiele für diese Gruppe der Zusatzstoffe sind Ethylenglycolmonoethylether und Diethylenglycolmonobutylether.
f) Wichtige Komponenten im Sinne der erfindungsgemäßen Einsatzgemische beziehungsweise Wertstoffe sind sogenannte kationische Wirkstoffkomponenten aus dem Gebiet der Netz-, Wasch- und Reinigungsmittel beziehungsweise der zugehörigen Hilfsstoffe. In Betracht kommen hier insbesondere die quartären Ammoniumverbindungen, die beispielsweise als Textilweichspülerkomponenten weit verbreitet sind und wenigstens einen, bevorzugt 2 längerkettige Alkylreste an einem quartären Stickstoffatom aufweisen.
   Besondere Bedeutung haben in diesem Zusammenhang heute die eine höhere ökologische Verträglichkeit aufweisenden sogenannten Esterquats, die - ausgehend von einem Trialkanolamin, üblicherweise Triethanolamin - längerkettige Fettsäurereste in Esterbindung enthalten. Üblicherweise liegen dabei Gemische von Mono-, Di- und Triestern vor, wobei beispielsweise der Anteil an Monoester 15 bis 25 Mol-%, der Gehalt an Diester 40 bis 50 Gew.-%, betragen und zum Rest Triester vorliegen kann. Zur einschlägigen Literatur wird beispielsweise verwiesen auf die EP-A 239 910 oder die DE-A 19 35 493. Esterquats der hier und in weiteren Literaturstellen - beispielsweise EP-A 0 293 953 und EP-A 0 309 052 - sind Umsetzungsprodukte aus Triethanolamin beziehungsweise ethoxyliertem Monoethanolamin mit Gemischen aus gesättigten und ungesättigten Fettsäuren der Kettenlängen C 6 bis C 28. Das dabei primär entstehende Reaktionsprodukt wird anschließend mit Dimethylsulfat quaterniert. Weiterführende Hinweise zu der hier betroffenen Stoffklasse finden sich in den Literaturstellen DE-A 37 10 064 (EP-A 0 284 036) und EP-A 0 295 385.

Die nachfolgenden Beispiele sollen den Gegenstand der Erfindung näher erläutern, ohne ihn darauf einzuschränken.

### Beispiele

### Beispiel 1

In einem Versuchssprühturm im Technikumsmaßstab vom Typ "Minor Production" der Firma Niro-Atomizer wurde ein Slurry des Natriumsalzes eines C₁₆/C₁₈-Fettalkoholsulfates unter Zusatz von Natriumcarbonat zu einem rieselfähigen Tensidpulver umgewandelt. Das Mischungsverhältnis zwischen dem Aniontensid und Natriumcarbonat betrug 4 : 1, bezogen auf Trockensubstanz.

Der eingesetzte wäßrige Slurry des Fettalkoholsulfates ist eine weiße bzw. schwach gelbe, feste Paste mit den Kenndaten:

| | |
|---|---|
| Waschaktive Substanz nach Epton | 54 - 58 % |
| Fettalkoholsulfat | 53 - 55 % |
| Unsulfiertes | 1 - 3 % |
| Gehalt an NaCl | 1 % |
| Gehalt an Na₂SO₄ | 2 % |
| pH-Wert (3%ige wäßrige Zubereitung) | 10 - 11,5 |

Nach Zugabe der wäßrigen Sodalösung stellte sich ein Trockensubstanzgehalt des Slurries von 47,7 Gew.-% ein. Der Slurry wird über eine 2-Stoffdüse (Treibgas Stickstoff) versprüht und im Gegenstrom mit überhitztem Wasserdampf getrocknet. Zur Heißdampftrocknung wurden die folgenden Betriebsparameter eingestellt:

| | |
|---|---|
| Dampfeintrittstemperatur | 250°C |
| Dampfaustrittstemperatur | 175 - 180°C |
| Turmunterdruck | 16 mbar |
| Feedpumpendruck | 5,5 bar |
| Feedtemperatur | 80°C |
| Feedmenge: | 12 kg/h |
| Dampfmenge: | 350 m³/h |
| Treibgas der Zweistoffdüse | |
| Menge: | 3,3 m³/h |
| Druck: | 0,2 bar |

Das erhaltene Produkt hatte einen Trockensubstanzgehalt von 99,2 Gew.-%. Das Schüttgewicht betrug 293 g/l. Eine 90%ige Löslichkeit in Wasser bei 20°C wurde bei 40 Sekunden ermittelt. Der Weißgrad des getrockneten Produktes erreicht bei einer Wellenlänge von 460 nm den Wert von 77,6. Im nachfolgenden wurde dem oben aufgeführten Slurryansatz ein Bleichmittel zugefügt. Die Betriebsparameter der Heißdampftrocknung sind mit den oben genannten identisch. Durch Zusatz von unterschiedlichen Mengen an Natriumhypochlorit als Bleichmittel konnten folgende Produktqualitäten erzielt werden:

| Zusammensetzung | NaOCl bzgl.TS (%) | Schüttgewicht (g/l) | Löslichkeit (90%) min/s | Weißgrad (460 nm ohne UV) |
|---|---|---|---|---|
| 80 % Sulfopon T55 | 0 | 293 | -/40 | 77,6 |
| 20 % Soda | | | | |
| | | | | |
| 80 % Sulfopon T55 | 0,5 | 226 | -/41 | 80,1 |
| 20 % Soda | | | | |
| | | | | |
| 80 % Sulfopon T55 | 1 | 230 | -/37 | 80,3 |
| 20 % Soda | | | | |
| "Sulfopon T55" Handelsbezeichnung der Anmelderin für ein Aniontensid auf Fettalkoholsulfat-Basis. | | | | |

Zum Vergleich zeigt ein Textilwaschmittel des Handels ("Persil TAS" der Anmelderin) einen Weißgrad von 79,7 im Wellenbereich von 460 nm. Die Betrachtung des gesamten Wellenbereiches durch Unterstützung mit UV-Licht ist bei den oben angeführten Aniontensidpulvern nicht relevant, da keine optischen Aufheller mitverwendet werden.

### Beispiel 2

Es wurde wie in Beispiel 1 gearbeitet. Aufgetrocknet wurde in der Phase des überhitzten Wasserdampfes ein Slurry mit 24,1% Feststoffgehalt. Die Feststoffbestandteile der Ausgangsmischung waren das in Beispiel 1 genannte Natriumsalz eines C_{16/18}-Fettalkoholsulfates ("Sulfopon T55"), das Di-Natriumsalz einer C_{16/18}-alpha-Sulfofettsäure und Natriumcarbonat im Mischungsverhältnis 4 : 1 : 1. Das getrocknete Pulver hatte ein Schüttgewicht von 220 g/l und eine Löslichkeit (90%) von 20 Sekunden.

Das Di-Natriumsalz der C_{16/18}-alpha-Sulfofettsäure ist eine schwach bräunliche Paste mit folgenden Kenndaten:

| | |
|---|---|
| Waschaktive Substanzen nach Epton | 26,3 % |
| unsulfonierte Fettsäure | 2,09 % |
| Sulfiergrad | 89,6 % |
| Gehalt an NaCl | 2,2 % |
| Gehalt an Na₂SO₄ | 3,1 % |

Die nachfolgende Tabelle enthält die Produkteigenschaften der heißdampfgetrockneten Pulver mit unterschiedlicher Bleichmittelzugabe im Ausgangsslurry:

**Tabelle 2:**

| Versuchsergebnisse | | | | |
|---|---|---|---|---|
| Zusammensetzung | NaOCl bzgl.TS (%) | Schüttgewicht (g/l) | Löslichkeit (90%) min/s | Weißgrad (460 nm ohne UV) |
| Sulfopon T55 + Soda + Disalz (4 : 1 : 1) | 0 | 220 | -/20 | 48,6 |
| | | | | |
| Sulfopon T55 + Soda + Disalz (4 : 1 : 1) | 1 | 249 | -/35 | 64,9 |
| | | | | |
| Sulfopon T55 + Soda + Disalz (4 : 1 : 1) | 2 | 248 | -/32 | 67,1 |
| | | | | |
| Sulfopon T55 + Soda + Disalz (4 : 1 : 1) | 4 | 253 | -/16 | 76,0 |

### Beispiel 3

Der in Beispiel 1 aufgeführte Slurry des Aniontensides auf Fettalkoholsulfatbasis mit Sodazusatz hat einen talgartig dumpfen Geruch, den man als unangenehm wahrnimmt. Insbesondere bei Erwärmung der Paste verstärkt sich die Geruchsbelästigung. Nach der Heißdampftrocknung, ausgeführt wie in Beispiel 1, ist das pulverförmige Produkt geruchslos. Nach der Feinstaubbindung durch Granulierung beliebiger Art erfolgt keinerlei Reizung der Schleimhäute.

### Beispiel 4

Das Tensidgemisch aus Beispiel 2, Natriumsalz eines C_{16/18}-Fettalkoholsulfates und Di-Natriumsalz einer C_{16/18}-alpha-Sulfofettsäure, hat im pastösen Ausgangszustand einen talgartig dumpfen Geruch, der besonders im wärmeren Zustand (T ~ 40°C) und bei längerer Lagerung als sehr aufdringlich und unangenehm empfunden wird. Bei dem mit Heißdampf getrockneten Produkt handelt es sich um ein lagerstabil geruchsneutrales Pulver, das vorbehaltlos akzeptiert wird.

## Patentansprüche

1. Verfahren zur Verbesserung des Reinheitsgrades und insbesondere der Beschaffenheit von Farbe und Geruch in Wertstoffen und Wertstoffgemischen aus dem Bereich der Netz-, Wasch- und/oder Reinigungsmittel (Einsatzgut), die auch in wäßriger Zubereitungsform vorliegen können, durch deren Behandlung mit einem Strom überhitzten Wasserdampfes bei gegebenenfalls gleichzeitiger Auftrocknung eines wasserhaltigen Einsatzgutes mittels Heißdampftrocknung dadurch gekennzeichnet, daß man ein mit Verunreinigungen belastetes Einsatzgut in feinverteilter Form und im Bereich des Normaldrucks in den Strom des überhitzten Wasserdampfs einträgt, gewünschtenfalls zur Ausbildung von Farbverbesserungen Bleichmittel mitverwendet und dabei diese Behandlung des verunreinigten Einsatzgutes in einer von dem überhitzten Wasserdampf durchströmten Sprühzone und/oder Wirbelschicht vornimmt.

2. Verfahren nach Anspruch 1, dadurch gekennzeichnet, daß man ein verunreinigtes Einsatzgut der Behandlung unterwirft, das wenigstens eine kurzfristige Temperaturerhöhung auf die Verdampfungstemperatur des Wassers unter Verfahrensbedingungen, insbesondere auf Temperaturen im Bereich von etwa 90 bis 110°C, schadlos übersteht.

3. Verfahren nach Ansprüchen 1 und 2, dadurch gekennzeichnet, daß ein - als wasserfreies Material - im Temperaturbereich von 90 bis 110°C flüssiges und/oder bevorzugt festes Einsatzgut, das insbesondere geruchlich und/oder farblich nicht befriedigt, dem Verfahren unterworfen wird.

4. Verfahren nach Ansprüchen 1 bis 3, dadurch gekennzeichnet, daß ein tensidische Komponenten und/oder verwandte Verbindungen, wie Textil-Weichmacher, enthaltendes Einsatzgut dem Verfahren unterworfen wird.

5. Verfahren nach Ansprüchen 1 bis 4, dadurch gekennzeichnet, daß ein Einsatzgut mit überhitztem Wasserdampf behandelt wird, das mit insbesondere Geruchsbildnern, Wertstoffbräunungen und/oder sonstigen Verunreinigungen belastete Aniontenside, Niotenside, zwitterionische Tensidverbindungen und/oder kationische Verbindungen wie Textilweichmacher enthält.

6. Verfahren nach Ansprüchen 1 bis 5, dadurch gekennzeichnet, daß zur Farbkorrektur dem Einsatzgut oxidativ oder reduktiv wirkende Bleichmittel wie Wasserstoffperoxid, Persalze, Hypochlorit oder Reduktionsmittel wie hypophosphorige Säure beziehungsweise deren Salze, insbesondere Alkali- und/oder Erdalkalisalze zugemischt werden.

7. Verfahren nach Ansprüchen 1 bis 6, dadurch gekennzeichnet, daß die Reinigung in Verbindung mit und bevorzugt gleichzeitig mit der Gewinnung wenigstens weitgehend getrockneter,insbesondere schütt- und rieselfähiger Wertstoffe und Wertstoffgemische aus deren wasserhaltigen Zubereitungen durchgeführt wird.

8. Verfahren nach Ansprüchen 1 bis 7, dadurch gekennzeichnet, daß mit Einsatztemperaturen des überhitzten Wasserdampfes im Bereich von etwa 100 bis 450°C, vorzugsweise im Bereich von etwa 115 bis 350°C und dabei im Bereich des Normaldrucks oder nur geringfügig erhöhter oder abgesenkter Drucke gearbeitet wird.

9. Verfahren nach Ansprüchen 1 bis 8, dadurch gekennzeichnet, daß fließfähige und versprühbare wäßrige Lösungen, Emulsionen und/oder Suspensionen des verunreinigten Einsatzgutes in feinverteilter Form der Behandlung mit dem überhitzten Wasserdampf unterworfen und dabei wenigstens anteilsweise getrocknet werden.

10. Verfahren nach Ansprüchen 1 bis 9, dadurch gekennzeichnet, daß Bleichmittel in Mengen bis etwa 10 Gew.-%, vorzugsweise in Mengen von etwa 0,5 bis 5 Gew.-% dem zu bleichenden Einsatzgut zugesetzt werden.

11. Verfahren nach Ansprüchen 1 bis 10, dadurch gekennzeichnet, daß mit einer Heißdampfphase gearbeitet wird, der sehr geringe Mengen an Ozon zugesetzt worden sind, wobei Ozonmengen nicht über etwa 1 ppm, vorzugsweise unterhalb 0,5 ppm und insbesondere im Bereich von etwa 0,05 bis 0,2 ppm - ppm bezogen jeweils auf die aus dem Heißdampfkreislauf ausgeschleuste Menge an Brüdenteilstrom - bevorzugt sind.

12. Anwendung des Verfahrens zur Trocknung wäßriger Zubereitungen von Wertstoffen und/oder Wertstoffgemischen aus dem Bereich der Netz-, Wasch- und/oder Reinigungsmittel in der Sprühzone und/-oder Wirbelschicht unter Einsatz von überhitztem Wasserdampf als Trocknungsgas zur gleichzeitigen Reinigung eines mit insbesondere wasserdampfflüchtigen Verunreinigungen beladenen Einsatzgutes.

13. Ausführungsform nach Anspruch 12, dadurch gekennzeichnet, daß die Reinigung mit einer gleichzeitig im behandelten Gut ausgelösten oxidativen oder reduktiven Bleiche unter Gewinnung hellfarbiger Wertstoffe beziehungsweise Wertstoffgemische verbunden wird.

## Claims

1. A process for improving the purity and, in particular, the colour and odour quality of useful materials or mixtures of useful materials from the field of wetting agents, detergents and/or cleaners (starting material), which may even be present in the form of aqueous preparations, by treatment thereof with a stream of superheated steam and - optionally - simultaneous drying of a water-containing starting material with superheated steam, characterized in that a fine-particle impurity-containing starting material is introduced at normal pressure into the stream of superheated steam, in that bleaching agents are optionally used to improve colour and in that this treatment of the impurity-containing staring material is carried out in a spray zone and/or fluidized bed through which the superheated steam flows.

2. A process as claimed in claim 1, characterized in that an impurity-containing starting material capable of withstanding at least a brief increase in temperature to the evaporation temperature of water under the process conditions, more particularly to temperatures of about 90 to 100°C, is subjected to the treatment.

3. A process as claimed in claims 1 and 2, characterized in that a starting material which - as a water-free material - is liquid and/or preferably solid at temperatures of 90 to 110°C and which, in particular, is unsatisfactory in its odour and/or colour quality is subjected to the process.

4. A process as claimed in claims 1 to 3, characterized in that a starting material containing surfactants and/or related compounds, such as fabric softeners, is subjected to the process.

5. A process as claimed in claims 1 to 4, characterized in that a starting material containing anionic surfactants, nonionic surfactants, zwitterionic surfactant compounds and/or cationic compounds, such as fabric softeners, contaminated in particular with odour-forming, discolouring and/or other impurities is treated with superheated steam.

6. A process as claimed in claims 1 to 5, characterized in that oxidative or reductive bleaching agents, such as hydrogen peroxide, per salts, hypochlorite, or reducing agents, such as hypophosphorous acid or salts thereof, more particularly alkali metal and/or alkaline earth metal salts, are added to the starting material for colour correction.

7. A process as claimed in claims 1 to 6, characterized in that purification is carried out in conjunction with and preferably at the same time as the recovery of at least substantially dried, more particularly pourable and free- flowing useful materials and mixtures thereof from the water-containing preparations.

8. A process as claimed in claims 1 to 7, characterized in that the treatment is carried out at temperatures of the superheated steam of about 100 to 450°C and preferably in the range from 115 to 350°C and under normal pressure or only slightly elevated or reduced pressures.

9. A process as claimed in claims 1 to 8, characterized in that flowable and sprayable aqueous solutions, emulsions and/or suspensions of the impurity-containing starting material are subjected to the treatment with superheated steam in fine-particle form and are at least partly dried in the process.

10. A process as claimed in claims 1 to 9, characterized in that bleaching agents are added to the starting material to be bleached in quantities of up to about 10% by weight and preferably in quantities of about 0.5 to 5% by weight.

11. A process as claimed in claims 1 to 10, characterized in that a superheated steam phase to which very small quantities of ozone have been added is used, quantities of ozone of not more than about 1 ppm, preferably below 0.5 ppm and more particularly in the range from about 0.05 to 0.2 ppm - ppm based on the amount of vapour removed as a side stream from the main steam circuit - being preferred.

12. The use of the process for drying aqueous preparations of useful materials and/or mixtures of useful materials from the field of wetting agents, detergents and/or cleaners in the spray zone and/or fluidized bed using superheated steam as the drying gas for simultaneously purifying a starting material laden in particular with steam-volatile impurities.

13. The use claimed in claim 12, characterized in that purification is combined with oxidative or reductive bleaching simultaneously initiated in the treated material to recover light-coloured useful materials or mixtures thereof.

## Revendications

1. Procédé d'amélioration du degré de pureté et, en particulier, de la constitution de la teinte et de l'odeur de matières utiles ou de valeur et de mélanges de matières utiles ou de valeur appartenant à la gamme des produits mouillants, ainsi que des produits de lavage et/ou de nettoyage (produit utilisé), qui peuvent également être présents sous forme de préparation aqueuse, par traitement de ceux-ci par un courant de vapeur d'eau surchauffée, un produit utilisé étant, le cas échéant, séché simultanément par traitement à la vapeur surchauffée, caractérisé en ce que l'on introduit dans le courant de la vapeur d'eau surchauffée un produit utilisé chargé d'impuretés sous forme finement dispersée et dans le domaine de la pression normale, en co-utilisant le cas échéant des agents de blanchiment pour l'induction d'améliorations de teinte, et que l'on exécute ce traitement de produit utilisé contaminé dans une zone de pulvérisation et/ou dans un lit fluidisé parcouru par la vapeur d'eau surchauffée.

2. Procédé selon la revendication 1, caractérisé en ce que l'on soumet au traitement une produit utilisé contaminé, qui supporte sans dommage au moins une hausse de brève durée de sa température à la température de vaporisation de l'eau, dans les conditions opératoires, en particulier à des températures comprises dans la plage d'environ 90 à 110 °C.

3. Procédé selon les revendications 1 et 2, caractérisé en ce que l'on soumet au procédé, comme matière anhydre, un produit utilisé liquide et/ou de préférence solide dans la plage de température de 90 à 110 °C, qui n'est pas satisfaisant, en particulier, par l'odeur et/ou par la teinte.

4. Procédé selon les revendications 1 à 3, caractérisé en ce que l'on soumet au procédé un produit utilisé renfermant des composants tensioactifs et/ou des composés apparentés, tels des assouplissants pour les textiles.

5. Procédé selon les revendications 1 à 4, caractérisé en ce que l'on traite par de la vapeur d'eau surchauffée un produit utilisé, qui renferme des tensioactifs anioniques, des surfactifs non ioniques, des composés tensioactifs zwitterioniques et/ou des composés cationiques, tels des assouplissants pour les textiles et qui est chargé, en particulier, de formateurs d'odeur, de brunissements de matières de valeur et/ou d'autres impuretés.

6. Procédé selon les revendications 1 à 5, caractérisé en ce que pour corriger la teinte, on ajoute au produit utilisé des agents de blanchiment à action oxydante ou réductrice, tels l'eau oxygénée, des persels, de l'hypochlorite ou des réducteurs, tels l'acide hypophosphoreux ou des sels de celui-ci, en particulier des sels de métaux alcalins et/ou alcalino-terreux.

7. Procédé selon les revendications 1 à 6, caractérisé en ce que l'on opère le nettoyage en relation avec et de préférence en même temps que l'extraction des matières de valeur ou des mélanges de matières de valeur, séchés au moins ans une large mesure, en particulier déversables et coulants, à partir de leurs préparations aqueuses.

8. Procédé selon les revendications 1 à 7, caractérisé en ce que l'on travaille avec des températures d'utilisation de la vapeur d'eau surchauffée comprises dans l'intervalle d'environ 100 à 450 °C, de préférence d'environ 115 à 350 °C, dans la plage de la pression normale ou de pressions seulement légèrement augmentées ou abaissées.

9. Procédé selon les revendications 1 à 8, caractérisé en ce que des solutions, des émulsions et/ou des suspensions aqueuses fluides et pulvérisables du produit utilisé contaminé sont soumises au traitement par la vapeur d'eau surchauffée sous forme finement dispersée, et sont ainsi séchées au moins partiellement.

10. Procédé selon les revendications 1 à 9, caractérisé en ce que des agents de blanchiment sont ajoutés au produit utilisé à blanchir en proportions pouvant atteindre environ 10 % en poids, de préférence en quantités d'environ 0,5 à 5 % en poids.

11. Procédé selon les revendications 1 à 10, caractérisé en ce que l'on travaille avec une phase de vapeur surchauffée, à laquelle de très faibles quantités d'ozone ont été ajoutées, des proportions d'ozone ne dépassant pas environ 1 ppm, de préférence inférieures à 0,5 ppm et, en particulier, situées dans l'intervalle d'environ 0,05 à 0,2 ppm (dans chaque cas par rapport à la quantité du courant partiel de buée soustraite au circuit de vapeur surchauffée) étant préférées.

12. Application du procédé pour le séchage de préparations aqueuses de matières utiles ou de valeur et/ou de mélanges de matières utiles ou de valeur, appartenant à la gamme des produits mouillants, ainsi que des produits de lavage et/ou de nettoyage, dans la zone de pulvérisation et/ou dans un lit fluidisé, en utilisant de la vapeur d'eau surchauffée comme gaz de séchage, pour l'épuration simultanée d'un produit utilisé chargé d'impuretés particulièrement entraînables à la vapeur.

13. Forme de réalisation selon la revendication 12, caractérisée en ce que l'épuration est liée à un blanchiment par oxydation ou par réduction déclenché simultanément dans la matière traitée, en obtenant ou en extrayant les matières de valeur ou les mélanges de matières de valeur de teinte claire.
